# FASCICULE DE BREVET EUROPEEN

(11) **EP 3 807 416 B1**
(45) Date de publication et mention de la délivrance du brevet: **13.04.2022**
(21) Numéro de dépôt: 19737863.1
(22) Date de dépôt: 13.06.2019
(51) Int. Cl.: C12Q 1/04, G01N 33/569

(54) **PROCÉDÉ DE DÉTECTION DES BACTÉRIES SELON LEUR SIGNAL GRAM DANS UN ÉCHANTILLON COMPLEXE**
VERFAHREN ZUM NACHWEIS VON BAKTERIEN JE NACH DEREN GRAMSIGNAL IN EINER KOMPLEXEN PROBE
METHOD FOR DETECTING BACTERIA ACCORDING TO THE GRAM SIGNAL THEREOF IN A COMPLEX SAMPLE

(30) Priorité: 18.06.2018 FR 1855350
(43) Date de publication de la demande: 21.04.2021
(73) Titulaire: Maat Pharma, 69007 Lyon (FR); Bioaster, 69007 Lyon (FR)
(72) Inventeur: DUQUENOY, Aurore, 69007 Lyon (FR); BELLAIS, Samuel, 69007 Lyon (FR); THOMAS, Vincent, 69007 Lyon (FR)
(74) Mandataire: Santarelli
(86) Numéro de dépôt international: PCT/FR2019/051435
(87) Numéro de publication internationale: WO 2019/243714

(56) Documents cités:
- S.N. LANGERHUUS ET AL: "Gram-typing of mastitis bacteria in milk samples using flow cytometry", JOURNAL OF DAIRY SCIENCE., vol. 96, no. 1, 1 janvier 2013 (2013-01-01), pages 267-277, XP055545668, US ISSN: 0022-0302, DOI: 10.3168/jds.2012-5813
- David J. Mason ET AL: "A Fluorescent Gram Stain for Flow Cytometry and Epifluorescence Microscopy", Applied and Environmental Microbiology, 1 juillet 1998 (1998-07-01), pages 2681-2685, XP055545478, United States Extrait de l'Internet: URL:https://aem.asm.org/content/aem/64/7/2 681.full.pdf
- R. SCHUCH ET AL: "Combination Therapy With Lysin CF-301 and Antibiotic Is Superior to Antibiotic Alone for Treating Methicillin-Resistant Staphylococcus aureus-Induced Murine Bacteremia", JOURNAL OF INFECTIOUS DISEASES. JID, vol. 209, no. 9, 28 novembre 2013 (2013-11-28), pages 1469-1478, XP055330867, US ISSN: 0022-1899, DOI: 10.1093/infdis/jit637
- PAULA YAGÜE ET AL: "Subcompartmentalization by cross-membranes during early growth of Streptomyces hyphae", NATURE COMMUNICATIONS, vol. 7, no. 1, 12 août 2016 (2016-08-12), XP055545860, DOI: 10.1038/ncomms12467
- Aurore Duquenoy ET AL: "Gram proportion determination in complex ecosystems using flow cytometry", , 20 juin 2018 (2018-06-20), XP055545648, Extrait de l'Internet: URL:https://www.maatpharma.com/docs/180528 _PosterAdebiotech.pdf [extrait le 2019-01-22]

## Description

La présente demande concerne un procédé de détection de la proportion de bactéries à Gram positif et de la proportion de bactéries à Gram négatif dans un échantillon, en particulier dans un échantillon complexe. La détection des bactéries se fait selon leur signal Gram.

Elle concerne également un kit de marquage des bactéries à Gram positif en cytométrie en flux, notamment pour des échantillons complexes comme des échantillons de microbiote - humain ou animal.

En particulier, l'invention se rapporte à l'utilisation d'un kit de marquage de bactéries, notamment dans des méthodes qui font l'objet de l'invention, en cytométrie en flux, alliant une lectine, la lectine de blé (en anglais, Wheat Germ Agglutinin ou WGA), couplée à un fluorochrome, avec un antibiotique, la vancomycine couplée à un fluorochrome émettant une fluorescence à une longueur d'onde différente de celle couplée au WGA. De manière optionnelle, le kit peut comprendre la vancomycine non-couplée à un fluorochrome (non-marquée) et/ou le chlorure de potassium. Le kit est utile pour la détection par fluorescence des bactéries à Gram positif, notamment par couplage des mesures de fluorescence relatives des deux fluorochromes différents. Ces mesures de fluorescence relatives permettent de distinguer des familles bactériennes à Gram positif entre elles. Elles permettent aussi de distinguer les genres bactériens entre eux. Elles permettent également de distinguer des espèces de bactéries à Gram positif telles que *Bacillus subtilis, Lactococcus lactis, Staphylococcus aureus, Enterococcus faecalis, et Lactobacillus spp.,* entre elles. Cette liste n'est pas exhaustive et de nombreuses espèces aérobies ou anaérobies pourraient être distinguées comme des *Clostridium* et *Faecalibacterium.*

Le microbiote intestinal humain, couramment appelé « flore intestinale », est l'ensemble des micro-organismes (bactéries, levures et champignons) qui se trouvent dans le système gastro-intestinal humain (estomac, intestin et côlon). La diversité bactérienne est estimée à l'heure actuelle à environ 10³ espèces bactériennes composant le microbiote intestinal dominant d'un individu adulte, avec une abondance de 10¹⁴ bactéries, représentant un métagénome bactérien de 200000 à 800000 gènes chez chaque individu, soit 10 à 50 fois le nombre de gènes du génome humain. Ainsi, le microbiote intestinal humain est un écosystème très diversifié, complexe et spécifique de chaque individu.

La cytométrie en flux représente une technique intéressante par rapport aux méthodes basées sur la culture pour étudier des écosystèmes complexes comme le microbiote intestinal. Initialement conçue pour l'analyse des cellules, la cytométrie en flux a été adaptée pour caractériser et dénombrer les populations microbiennes. Elle est notamment utilisée pour de la caractérisation microbienne comme la détermination de la sensibilité aux antibiotiques [Saint-Ruf, C., et al., Antibiotic Susceptibility Testing of the Gram-Negative Bacteria Based on Flow Cytometry. Frontiers in Microbiology, 2016. 7 et Pereira, P.M., et al., Fluorescence ratio imaging microscopy shows decreased access of vancomycin to cell wall synthetic sites in vancomycin-resistant Staphylococcus aureus. Antimicrob Agents Chemother, 2007. 51(10) : p. 3627-33], l'analyse de la différenciation pendant la formation de biofilms, l'évaluation de la viabilité des populations ou encore la différenciation de bactéries à Gram positif et à Gram négatif. En cytométrie en flux, la discrimination entre les bactéries à Gram positif et celles à Gram négatif est différente de la technique classique de coloration Gram qui se base uniquement sur la présence ou absence de la membrane externe. En cytométrie, la discrimination peut se faire via la présence de structures plus ou moins exprimées à la surface des bactéries ou plus ou moins accessibles. Ainsi, ce ciblage permet parfois d'observer une discrimination plus précise des populations bactériennes.

La détermination du statut Gram des populations microbiennes présentes dans des échantillons complexes peut s'effectuer avec des colorations utilisant le violet de gentiane et la safranine ou la fuchsine, par exemple. Les suspensions bactériennes doivent être fixées sur lame avant de pouvoir réaliser la coloration qui alterne les différents colorants, fixateurs, décolorants ainsi que les étapes de lavage et séchage. Ces techniques peuvent donc être chronophages et les résultats obtenus peuvent être variables. En effet, selon les décolorants utilisés, souvent l'acétone et l'éthanol, et les temps de pose, les résultats d'identification peuvent être différents et peuvent induire une mauvaise interprétation du statut Gram de l'échantillon analysé. Afin d'assurer la reproductibilité des résultats, de nouvelles techniques d'identification du statut Gram ont été développées. Cela induit l'utilisation de marqueurs fluorescents pour réaliser des analyses en microscopie ou de plus en plus en cytométrie en flux.

Le brevet WO 02/057482 décrit l'utilisation de la lectine WGA labélisée avec le fluorochrome Oregon Green^{®} en combinaison avec un marqueur de l'ADN. Cette combinaison permet de déterminer le pourcentage de bactéries à Gram positif et à Gram négatif dans un mélange de culture pure de différentes bactéries ou d'un échantillon de lait. La lectine WGA couplée à un autre fluorochrome, la fluorescéine isothiocyanate, a aussi été utilisée pour analyser des bactéries acidophiles et archées [FIFE, D.J., et al., Evaluation of a Fluorescent Lectin-Based Staining Technique for Some Acidophilic Mining Bacteria. Appl Environ Microbiol, 2000. 66(5): p. 2208-2210]. L'utilisation de la lectine WGA couplé à un fluorochrome dans une méthode pour différencier la proportion de bactéries gram-positives et gram-négatives est décrit par Langerhuus et al. (Journal of Dairy science 2013).

Un des désavantages de l'utilisation du WGA réside dans sa capacité à se fixer sur des structures particulières présentes chez les bactéries à Gram positif, mais non forcément spécifiques de ces bactéries. Certaines des structures reconnues par le WGA peuvent aussi être retrouvées chez des bactéries à Gram négatif.

Des méthodes et le kit de la présente invention ont été développés pour permettre une discrimination plus précise entre les bactéries à Gram positif et à Gram négatif, indépendamment de la viabilité de l'échantillon analysé et indépendamment du fait que les bactéries soient aérobies ou anaérobies. Les méthodes sont applicables sur des échantillons complexes comme des échantillons de microbiote. Elles sont également applicables sur des échantillons de cultures bactériennes (non complexes).

### Sommaire de l'invention

La présente invention concerne un procédé et l'utilisation d'un kit telle que revendiqué.

### Description des Figures

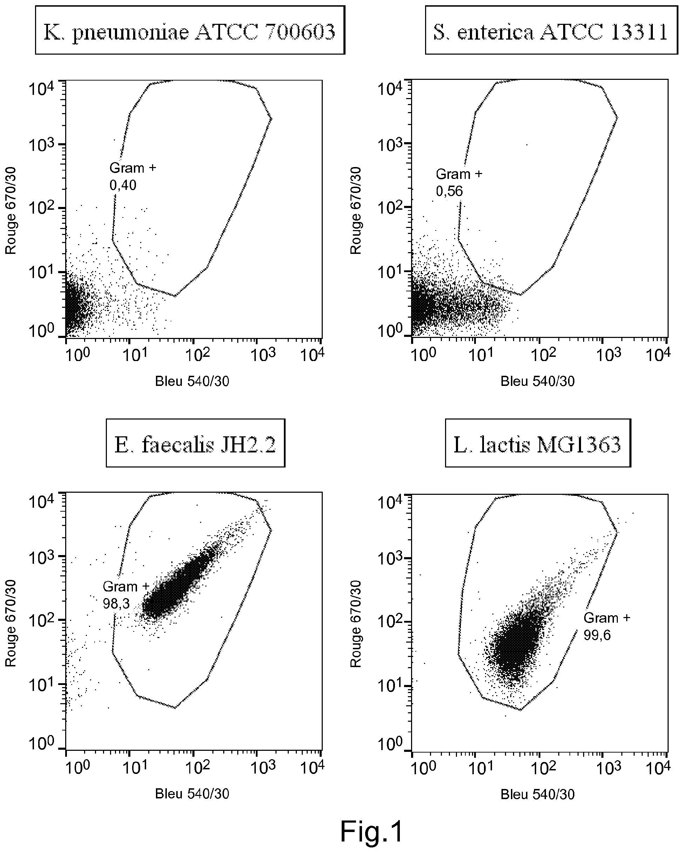

La Figure 1 : Des résultats de cytométrie en flux de l'Exemple 1. L'axe X représente l'intensité de la fluorescence à une longueur d'onde de 540nm ± 15nm, qui correspond à la fluorescence de la vancomycine couplée à Bodipy^{®} FL. L'axe Y représente l'intensité de la fluorescence à une longueur d'onde de 670nm ± 15nm, qui correspond à la fluorescence de WGA couplé à Alexa Fluor^{®}647.

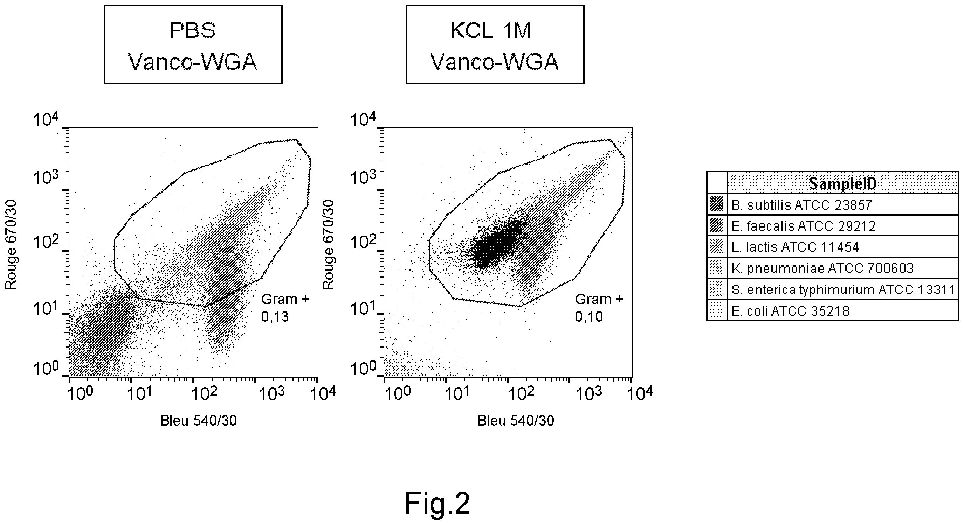

La Figure 2 : Des résultats de cytométrie en flux de l'Exemple 2. L'axe X représente l'intensité de la fluorescence à une longueur d'onde de 540nm ± 15nm, qui correspond à la fluorescence de la vancomycine couplée à Bodipy^{®}FL. L'axe Y représente l'intensité de la fluorescence à une longueur d'onde de 670nm ± 15nm, qui correspond à la fluorescence de WGA couplé à Alexa Fluor^{®} 647.

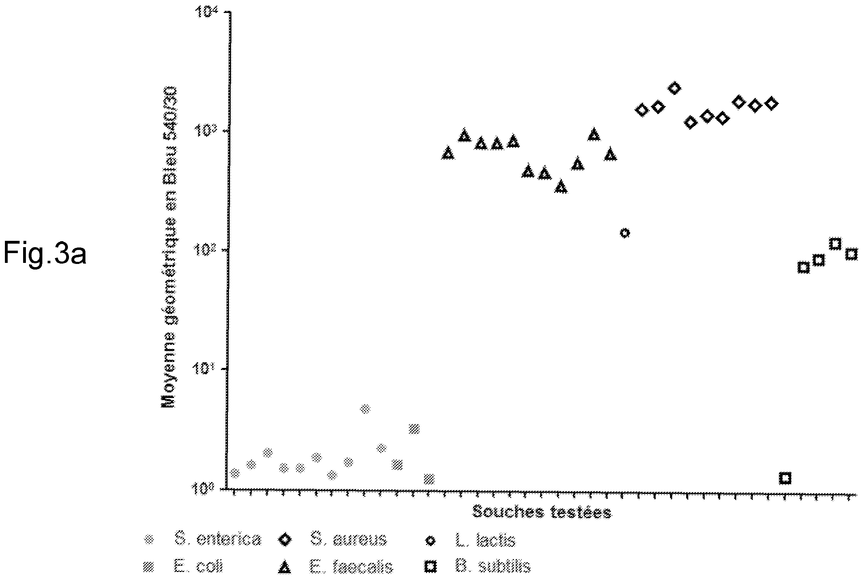

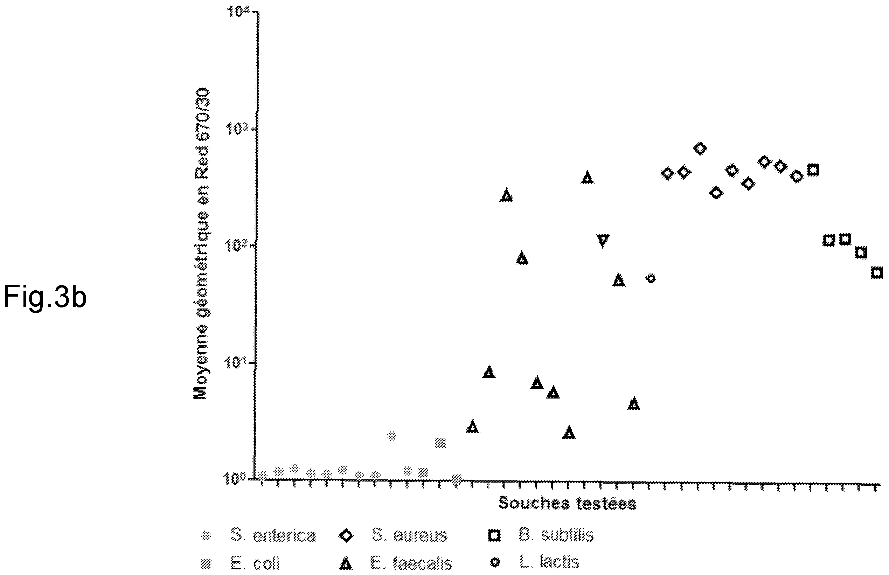

Les Figures 3A et 3B: Des résultats de l'expérience décrite à l'Exemple 3. La Figure 3A montre la moyenne géométrique des fluorescences obtenues, pour les différentes espèces testées, à une longueur d'onde de 540nm ± 15nm, qui correspond à la fluorescence de la vancomycine couplée à Bodipy^{®} FL. Le graphique de la Figure 3B montre la moyenne géométrique des fluorescences obtenues, pour les différentes espèces testées, à une longueur d'onde de 670nm ± 15nm, qui correspond à la fluorescence de WGA couplé à Alexa Fluor^{®} 647^{®}.

### Description Détaillée

Les inventeurs en cherchant des moyens de déterminer avec plus de précision des bactéries à Gram positif ont développé un kit et un procédé basés sur la combinaison d'une lectine, le WGA, et d'un antibiotique, la vancomycine, couplés aux fluorochromes émettant de la fluorescence à des longueurs d'onde différentes. Cette combinaison permet d'analyser en cytométrie en flux la proportion de bactéries à Gram positif et à Gram négatif dans des échantillons complexes, tels que des échantillons de microbiote.

Par « bactéries à Gram positif » on entend des bactéries qui sont mises en évidence par le marquage de Gram (le violet de gentiane). Par « bactéries à Gram négatif» on entend des bactéries qui ne sont pas mises en évidence par le marquage de Gram.

La vancomycine volumineuse (1 500 à 2 000 daltons) est un glycopeptide qui inhibe la synthèse du peptidoglycane des bactéries en se fixant sur les chaines D-Ala-D-Ala terminales. Ces structures sont aussi retrouvées chez les bactéries à Gram négatif mais ne sont pas accessibles par la vancomycine de par la présence de la membrane externe. En outre, la vancomycine est trop volumineuse pour emprunter les pores de la membrane extérieure des bactéries à Gram négatif. Par contre, la vancomycine ne se fixe pas sur les bactéries résistantes qui ne possèdent pas cette chaine D-Ala-D-Ala et par conséquent ne marque pas ces dernières.

Le WGA n'est pas, à la différence de la vancomycine, spécifique des bactéries à Gram positif. Il se fixe préférentiellement et différentiellement sur ces bactéries grâce à leur reconnaissance de structures plus ou moins présentes chez les bactéries à Gram positif, l'acide sialique et le N-acétyl-D-glucosamine. Nous pouvons donner comme exemple le *Streptococcus agalactiae,* plus connu sous le nom de *Streptococcus* B (SGB) qui possède beaucoup d'acide sialique dans sa paroi induisant un marquage important par le WGA.

L'ajout de WGA en plus de la vancomycine permet de renforcer l'identification de bactéries à Gram positif qui pourraient être résistantes à la vancomycine et donc non identifiées avec uniquement cet antibiotique. Ainsi, cette combinaison de ces deux marqueurs - vancomycine couplée au premier fluorochrome et WGA couplé au deuxième fluorochrome -représente une combinaison intéressante non seulement pour discriminer les populations à Gram positif et à Gram négatif, mais également pour pouvoir générer un signal fluorescent signature des espèces de bactéries à Gram positif présentes dans l'échantillon. Ces informations permettent de caractériser des populations bactériennes complexes, telles que celles typiques des échantillons de microbiote, notamment des échantillons humain et animal.

Des exemples de fluorochromes incluent, mais ne sont pas limités à des agents de marquage Alexa Fluor^{®}, BODIPY^{®}, fluorescéine ou l'un de ses dérivés, sels ou esters, des agents de marquage Oregon Green^{®} 488 et Oregon Green^{®} 514, des agents de marquage Rhodamine Green et Rhodamine Green-X, éosine, tétraméthylrhodamine, des agents de marquage Lissamine Rhodamine B et Rhodamine Red-X, X-Rhodamine, des agents de marquage Texas Red^{®} et Texas Red^{®}-X, naphthofluorescéine ou l'un de ses dérivés, sels ou esters, Carboxyrhodamine 6G, des agents de marquage QSY : désactivateurs de fluorescence, vert de malachite non fluorescent, dérivés de coumarine, l'agent de marquage Pacific Orange, Cascade Blue et autres dérivés pyrène, Cascade Yellow et autres dérivés pyridyloxazole, des naphtalènes (par exemple, chlorure de dansyle), l'agent de marquage dapoxyl, bimane, 1-diméthylamine-N(2-azidoéthyl) naphthalène-5 -sulfonamide, 6-(6-amino-2-(2-azidoéthyl)1,3-dioxo-1H-benzo(de)-2(3H)isoquinoléine, 6-(6-amino-2-(2-propinyl)1,3-dioxo-1H-benzo(de)-2(3H)isoquinoléine, 8-(4-azidoéthyloxyphényl)-2,6-diéthyl-1,3,5,7-tétraméthyl-4,4-difluoro-4-bora-3a,4a-diaza-s-indacène, 8-(4-propynyloxyphényl)-2,6-diéthyl-1,3,5,7-tétraméthyl-4,4-difluoro-4-bora-3a,4a-diaza-s-indacène, 1-(3-azido-propoxy)-7-méthylamino-phénoxazine-3-one, 1-(2-propynyl)-7-méthylamino-phénoxazine-3-one, chlorure de N-(5-(3-azidopropylamino)-9H-benzo(a)-phénoxazine-9-ylidéne)-N-méthyl-méthanaminium, chlorure de N-(5-(3-propynyl-amino)-9H-benzo(a)-phénoxazine-9-ylidène)-N-méthyl-méthanaminium, perchlorate de (9-(3-azido-propoxy)-7-pipéridine-1-yl-phénoxazine-3-ylidène)-diméthyl-ammonium. On peut également citer (les) dérivé(s) de 4-méthylumbelliférone, 1-(2-benzoylphényl)-6-chloro-1H-indol-3-yl-bêta-glucopyranoside; 1-(2-benzoylphényl)-6-chloro-1H-indol-3-yl-bêta-galactopyranoside; 1-(2-benzoylphényl)-6-chloro-1H-indol-3-yl-acétate; 1-(2-benzoylphényl)-6-chloro-1H-indol-3-yl-phosphate, l'acide 8-hydroxypyrène-1,3,6-trisulfonique (HPTS), un composé cyanine ou l'un de ses dérivés. Voir également, Kele et al. (2009) Org. Biomol. Chem. 7:3486-3490; Nagy et al. (2010) Chem. Asian J. 5:773-777; Filnov et al. (2011) Nat. Biotechnol. 29:757-761; Subach et al. (2011) Nature Methods 8:771-777; Yang et al. (2011) J. Am. Chem. Soc. 133:9964- 9967; Zin (2011) Nature Methods 8:726-728; et "Fluorophores and their amine- reactive derivatives," Chapitre 1.

En tant que fluorochromes préférés pour utilisation dans l'invention, on peut citer les fluorochromes dans le **Tableau 1** ci-dessous :

### Fluorochromes conjugués

| **Fluorochrome (nom)** | **λ excitation (nm)** | **λ émission (nm)** | **PM** | **Notes** | **Numéro CAS** |
|---|---|---|---|---|---|
| Hydroxycoumarine | 325 | 386 | 331 | Ester de Succinimidyle | 43070-85-5 |
| Aminocoumarine | 350 | 445 | 330 | Ester de Succinimidyle | 30230-57-0 |
| Méthoxycoumarine | 360 | 410 | 317 | Ester de Succinimidyle | |
| Cascade Blue | (375) ; 401 | 423 | 596 | Hydrazide | |
| Pacific Blue | 403 | 455 | 406 | Maléimide | |
| Pacific Orange | 403 | 551 | | | 1122414-42 -9 |
| Lucifer Yellow | 425 | 528 | | | 82446-52-4 |
| NBD | 466 | 539 | 294 | NBD-X | |
| R-Phycoerythrin (PE) | 480 ; 565 | 578 | 240 k | | 11016-17-4 |
| PE-Cy5 conjugates | 480; 565 ; 650 | 670 | | aka Cychrome, R670, Tri-Color, Quantum Red | |
| PE-Cy7 conjugates | 480 ; 565 ; 743 | 767 | | | |
| Red 613 | 480 ; 565 | 613 | | PE-Texas Red | |
| PerCP | 490 | 675 | | Protéine de chlorophylle de péridinine | |
| TruRed | 490, 675 | 695 | | PerCP-Cy5.5 conjugate | 396076-95-2 |
| FluorX | 494 | 520 | 587 | (GE Healthcare) | |
| Fluorescéine | 495 | 519 | 389 | FITC; sensible au pH | 2321-07-5 |
| BODIPY^{®} FL | 503 | 512 | | | 165599--63--3 |
| TRITC | 547 | 572 | 444 | TRITC | 107347-53-5 |
| X-Rhodamine | 570 | 576 | 548 | XRITC | |
| Lissamine Rhodamine B | 570 | 590 | | | 3520-42-1 |
| Texas Red | 589 | 615 | 625 | Chlorure de Sulfonyle | 82354-19-6 |
| Allophycocyanin (APC) | 650 | 660 | 104 k | | 874103-50-1,874103-51-2 |
| APC-Cy7 conjugates | 650; 755 | 767 | | PharRed | |

### Marqueurs Alexa Fluor^{®}

| **Fluorochrome** | **λ excitation (nm)** | **λ émission (nm)** | **PM** | **Désactivateur** | **Numéro CAS** |
|---|---|---|---|---|---|
| Alexa Fluor^{®} 350 | 343 | 442 | 410 | | 244636-14-4 |
| Alexa Fluor^{®} 405 | 401 | 421 | 1028 | | 791637-08-6 |
| Alexa Fluor^{®} 430 | 434 | 540 | 702 | | |
| Alexa Fluor^{®} 488 | 499 | 519 | 643 | QY 0.92 | 247144-99-6 |
| Alexa Fluor^{®} 500 | 503 | 525 | 700 | | 798557-08-1 |
| Alexa Fluor^{®} 514 | 517 | 542 | 714 | | 798557-07-0 |
| Alexa Fluor^{®} 532 | 530 | 555 | 724 | QY 0.61 | 247145-11-5 |
| Alexa Fluor^{®} 546 | 561 | 572 | 1079 | QY 0.79 | 2471.45-23-9 |
| Alexa Fluor^{®} 555 | 553 | 568 | 1250 | OY 0.1 | 644990-77-2 |
| Alexa Fluor^{®} 568 | 579 | 603 | 792 | QY 0.69 | 247145-38-6 |
| Alexa Fluor^{®} 594 | 591 | 618 | 820 | QY 0.66 | 2471.45-86-4 |
| Alexa Fluor^{®} 610 | 610 | 629 | 1285 | | 900528-62-3 |
| Alexa Fluor^{®} 633 | 632 | 648 | 1200 | | 477780-06-6 |
| Alexa Fluor^{®} 647 | 652 | 668 | 1300 | QY 0.33 | 400051-23-2 |
| Alexa Fluor^{®} 660 | 663 | 691 | 1100 | | 422309-89-5 |
| Alexa Fluor^{®} 680 | 680 | 702 | 1150 | | 422309-67-9 |
| Alexa Fluor^{®} 700 | 696 | 719 | 1400 | | 697795-05-4 |
| Alexa Fluor^{®} 750 | 752 | 776 | 1300 | | 697795-06-5 |
| Alexa Fluor^{®} 790 | 782 | 804 | 1750 | | 950891-33-5 |

Les structures de plusieurs marqueurs Alexa Fluor^{®} sont montrées ci-dessous :

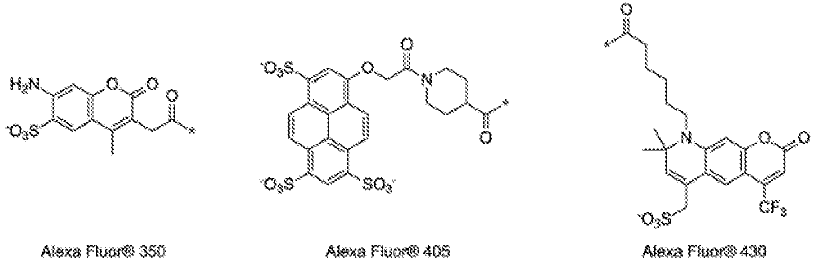

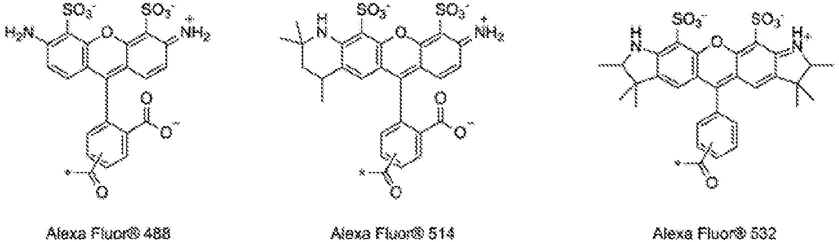

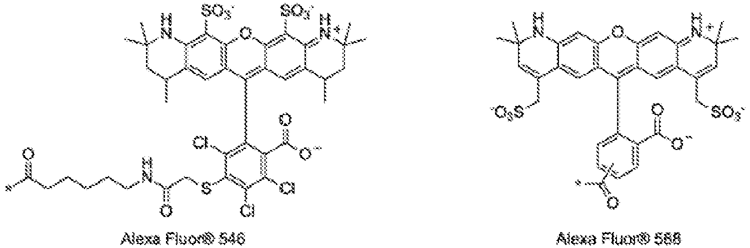

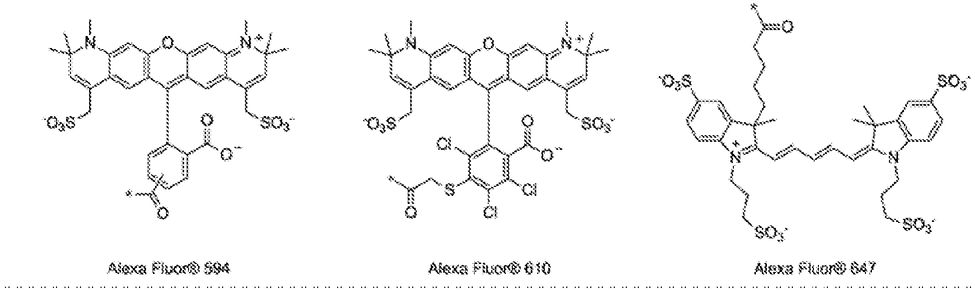

### Marqueurs Cy

| **Fluorochrome** | **λ excitation (nm)** | **λ émission (nm)** | **PM** | **Désactivateur** |
|---|---|---|---|---|
| Cy2 | 489 | 506 | 714 | QY 0.12 |
| Cy3 | (512) ; 550 | 570; (615) | 767 | QY 0.15 |
| Cy3B | 558 | 572; (620) | 658 | QY 0.67 |
| Cy3.5 | 581 | 594; (640) | 1102 | QY 0.15 |
| Cy5 | (625) ; 650 | 670 | 792 | QY 0.28 |
| Cy5.5 | 675 | 694 | 1128 | QY 0.23 |
| Cy7 | 743 | 767 | 818 | QY 0.28 |

### Marqueurs CF

Voir, par exemple, le site :
https://biotium.com/product/wheat-germ-agglutinin-wga-cf-dye-conjugates/

| **Conjugaison** | **Ex/Em** |
|---|---|
| CF^{®}350 | 347/448 nm |
| CF^{®}405S | 404/431 nm |
| CF^{®}405M | 408/452 nm |
| CF^{®}488A | 490/515 nm |
| CF^{®}532 | 527/558 nm |
| CF^{®}555 | 555/565 nm |
| CF^{®}568 | 562/583 nm |
| CF^{®}594 | 593/614 nm |
| CF^{®}633 | 630/650 nm |
| CF^{®}640R | 642/662 nm |
| CF^{®}680 | 681/698 nm |
| CF^{®}680R | 680/701 nm |
| CF^{®}770 | 770/797 nm |

Selon un mode de réalisation de l'invention, le fluorochrome couplé au WGA est choisi parmi les marqueurs CF^{®} du fournisseur Biotium (CA, Etats Unis) (voir le tableau « Marqueurs CF » ci-dessus).

Selon un mode de réalisation de l'invention, le fluorochrome couplé au WGA est choisi parmi les marqueurs du fournisseur Invitrogen/Molecular Probes (Carlsbad, CA, Etats Unis) comme la tétraméthylrhodamine, le Texas Red-X, Qdot 655, Oregon Green 488.

Selon un mode de réalisation de l'invention, le fluorochrome couplé au WGA est choisi parmi les fluorescéines, notamment la fluorescéine isothiocyanate-5 (numéro de CAS 3326-32-7), mieux connue de l'homme du métier par son nom commercial, FITC. Comme exemple de fournisseur, on peut citer MP Biomedicals (Santa Ana, CA, Etats Unis).

Selon un mode de réalisation de l'invention, le fluorochrome couplé au WGA est Alexa Fluor^{®} 647 (numéro de CAS 400051-23-2). Il est facilement disponible dans le commerce chez plusieurs fournisseurs. Comme exemple de fournisseurs, on peut citer Invitrogen/Molecular Probes (Carlsbad, CA, Etats Unis).

La structure chimique d'Alexa Fluor^{®} 647 est :

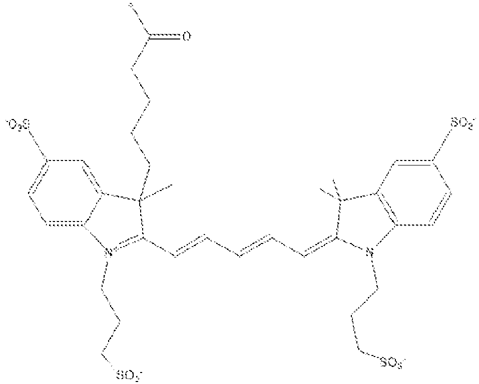

Selon un mode de réalisation de l'invention, le fluorochrome couplé à la vancomycine est choisi parmi l'acide propénoïque 3-BODIPY, ou un de ses dérivés, sels ou esters, les fluorescéines ou un de ses dérivés, sels ou esters, notamment la fluorescéine isothiocyanate-5 (numéro de CAS 3326-32-7), mieux connue de l'homme du métier par son nom commercial, FITC. Comme exemple de fournisseur, on peut citer Invitrogen/Molecular probes (Carlsbad, CA États-Unis).

Selon un mode de réalisation de l'invention, le fluorochrome couplé à la vancomycine est choisi parmi l'acide propénoïque 3-BODIPY (numéro de CAS 165500-63-3), mieux connu de l'homme du métier par son nom commercial, BODIPY FL^{®}, ou un de ses dérivés, sels ou esters. On peut citer des esters isothiocyanate, succinimidyle et succinimidyle sulfoné en tant qu'esters. Le nom BODIPY correspond à bore-dipyrométhene. Le nom IUPAC pour le corps BODIPY est 4,4-difluoro-4-bora-3a,4a-diaza-s-indacène.

Selon un mode de réalisation de l'invention, l'acide propénoïque 3-BODIPY (BODIPY FL^{®}) est le fluorochrome particulièrement préféré pour être couplé à la vancomycine. Le BODIPYFL^{®} est disponible dans le commerce. Comme exemple de fournisseurs, on peut citer Invitrogen/Molecular probes (Carlsbad, CA États-Unis).

La structure chimique du BODIPY FL^{®} est montrée ci-dessous :

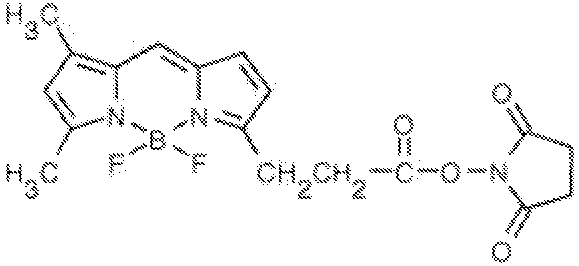

Selon un mode de réalisation préférée de l'invention, le WGA est couplé au fluorochrome Alexa Fluor^{®} 647 et la vancomycine est couplée au BODIPY FL^{®}.

Le WGA est disponible dans le commerce. Par exemple, on peut citer Sigma Aldrich (Saint-Louis, MO, États-Unis) et MP Biomedicals (Santa Ana, CA, Etats Unis). Le WGA est normalement fourni sous forme de lyophilisat prêt à être reconstitué avec de l'eau ou un autre liquide approprié, de préférence une solution aqueuse. Le couplage du WGA avec un fluorochrome se fait selon des méthodes connues de I'homme de métier. Un certain nombre de WGA-fluorochromes, comme les WGA-fluorochromes Alexa Fluor^{®}, sont disponibles dans le commerce. Par exemple, on peut citer les fournisseurs suivants : Invitrogen/Molecular probes (Carlsbad, CA Etats-Unis), Sigma Aldrich (Saint-Louis, MO, États-Unis) et Biotium (CA, Etats Unis).

La vancomycine est disponible dans le commerce, par exemple on peut citer Sigma Aldrich (Saint-Louis, MO, États-Unis) et Merck Millipore (Burlington, MA, États-Unis) en tant que fournisseurs. La vancomycine peut être fournie sous forme de poudre prête à être reconstituée avec de l'eau ou une solution aqueuse appropriée. Elle peut également être fournie en tant que solution. Le couplage de la vancomycine avec un fluorochrome se fait selon des méthodes connues de l'homme de métier. Un certain nombre de vancomycine-fluorochromes sont disponibles dans le commerce. Par exemple, on peut citer Invitrogen/Molecular probes (Carlsbad, CA Etats-Unis) en tant que fournisseur.

Les inventeurs ont observé que l'ajout de chlorure de potassium (KCI) à une concentration entre 0,3M et 3M permet une amélioration du marquage WGA pour certaines bactéries à Gram positif, par exemple, *Enterococcus faecalis* et *Lactococcus lactis,* et une augmentation du marquage WGA et de la vancomycine pour d'autres bactéries à Gram positif, par exemple, de *Bacillus subtilis,* toutefois sans augmenter le marquage des bactéries à Gram négatif. Ainsi, selon un mode de réalisation de l'invention, le marquage des bactéries de l'échantillon à analyser comprend une étape d'incubation avec du KCI à une concentration finale entre 0,3M et 3M, de préférence 1M. De préférence, l'incubation avec le KCI a lieu en même temps que l'incubation avec les agents de marquage, le WGA couplé à un fluorochrome et la vancomycine couplée à un fluorochrome.

Les inventeurs ont aussi confirmé que l'ajout de la vancomycine non couplée à un fluorochrome (la vancomycine non-marquée) améliore le marquage de certaines souches, comme pour *E. faecalis.* Il est connu, par exemple, que la vancomycine Bodipy^{®} FL se fixe difficilement sur *B. subtilis* et que l'ajout de vancomycine non-marquée en plus de la vancomycine Bodipy^{®} FL pourrait améliorer ce marquage.

Ainsi, selon un mode de réalisation de l'invention, le marquage des bactéries de l'échantillon à analyser peut se faire avec un mélange de la vancomycine non-marquée et de la vancomycine couplée à un fluorochrome. Le ratio entre la vancomycine non-marquée et la vancomycine couplée à un fluorochrome peut être entre 0,3 et 2,4, de préférence entre 0,75 et 1,25, plus préférentiellement 1.

Les inventeurs ont ainsi développé un kit pour la détection dans un échantillon contenant des bactéries, de la proportion de bactéries à Gram positif et de la proportion de bactéries à Gram négatif. De manière générale, le kit est mis en œuvre dans des méthodes de cytométrie en flux.

De manière générale, le kit de marquage des bactéries à Gram positif comprend :
i. la lectine du blé (le WGA), couplée à un fluorochrome,
ii. la vancomycine couplée à un fluorochrome émettant une fluorescence à une longueur d'onde différente de celle couplée au WGA et
iii. optionnellement la vancomycine non-marquée,
iv. optionnellement du chlorure de potassium (KCI).

Selon un mode de réalisation de l'invention, le kit de marquage des bactéries à Gram positif comprend :
i. un lyophilisat ou une solution de la lectine du blé (le WGA), couplée à un fluorochrome,
ii. une poudre ou solution de la vancomycine couplée à un fluorochrome émettant une fluorescence à une longueur d'onde différente de celle couplée au WGA,
iii. optionnellement la vancomycine non marquée sous forme de poudre ou solution,
iv. optionnellement du chlorure de potassium, sous forme de poudre ou solution.

Selon un mode de réalisation de l'invention, le kit de marquage des bactéries à Gram positif comprend :
i. un lyophilisat ou une solution de la lectine du blé (le WGA), couplée à Alexa Fluor^{®} 647 (numéro de CAS 400051-23-2),
ii. une poudre ou solution de la vancomycine, couplée à l'acide propénoïque 3-BODIPY (numéro de CAS 165500-63-3), mieux connu de l'homme du métier par son nom commercial, BODIPY^{®}FL,
iii. optionnellement la vancomycine (non marquée),
iv. optionnellement du chlorure de potassium (KCI).

Selon un mode de réalisation de l'invention, dans le kit, la solution de vancomycine couplée à BODIPY^{®}FL a une concentration de 0,2 à 4 µg/ml.

Selon un mode de réalisation de l'invention, dans le kit, la solution de la lectine du blé (le WGA), couplée à Alexa Fluor^{®} 647 (numéro de CAS 165500-63-3), a une concentration de 5 à 100 µg/ml.

L'invention concerne également un procédé de détection dans un échantillon contenant des bactéries, de la proportion de bactéries à Gram positif et de la proportion des bactéries à Gram négatif. Le kit décrit ci-dessus est conçu pour mettre en œuvre la méthode de l'invention. De manière générale, le procédé comprend les étapes :
a) incubation de l'échantillon contenant des bactéries, simultanément ou séquentiellement, avec
   - la lectine du blé (le WGA), couplée à un fluorochrome,
   - la vancomycine couplée à un fluorochrome émettant une fluorescence à une longueur d'onde différente de celle couplée au WGA,
   - optionnellement de la vancomycine,
   - optionnellement du chlorure de potassium,
b) centrifugation de l'échantillon,
c) optionnellement lavage de l'échantillon,
d) mesure de la fluorescence de l'échantillon.

Typiquement, l'étape d) est effectuée avec un cytomètre en flux aux longueurs d'ondes appropriées selon les fluorochromes choisis. En général, l'étape d) est effectuée en moins de 40 minutes, de préférence en moins de 30 minutes après la fin de l'étape c).

Selon un mode de réalisation de l'invention, le procédé peut comprendre une étape de fixation, qui peut avoir lieu avant, ou après, le marquage. Selon un mode de réalisation de l'invention, l'étape de fixation peut avoir lieu entre l'étape c) et l'étape d). Cette étape de fixation stabilise la dispersion de la lumière et le marquage de l'échantillon jusqu'à environ une semaine si l'échantillon est gardé à environ 4°C dans le noir.

Par exemple, l'échantillon peut être incubé avec du para-formaldéhyde (PFA) à une concentration, par exemple de 1% (v/v) pour une période d'environ 30 minutes à une heure trente minutes, à température ambiante par exemple. L'homme de métier sait adapter des conditions de cette étape de fixation. Si une étape de fixation a lieu, elle est généralement suivie d'au moins une, de préférence deux centrifugations, de 3200g à 15 000g pendant 5-20 minutes pour enlever tout résidu de PFA. L'étape de fixation permet ainsi d'étendre le délai entre la fin du marquage (étape c)) et l'étape d) et permet d'être flexible dans la planification du temps de manipulation.

Selon un mode de réalisation de l'invention, l'étape de fixation peut aussi avoir lieu avant l'étape a). Dans ce cas, on peut « fixer » l'état des cellules à analyser avant de procéder au marquage (l'étape a)). Cela permet de prolonger le temps avant marquage sans modification significative de l'état cellulaire.

Selon un mode de réalisation de l'invention, l'échantillon de cellules de l'étape a) a une concentration de 10⁴ à 10⁹ cellules/ml, de préférence de 10⁵ à 10⁷ cellules/ml. Le WGA couplé à un fluorochrome, la vancomycine couplée à un fluorochrome, éventuellement la vancomycine non marquée, et éventuellement du KCI, peuvent être ajoutés à l'échantillon cellulaire l'un après l'autre, ou simultanément. De préférence, ils sont ajoutés simultanément. Typiquement, la solution de marquage est préparée avec tous les marqueurs, éventuellement incluant la vancomycine non marquée et le KCL.

Selon un mode de réalisation de l'invention, la concentration finale dans l'échantillon de WGA couplé à un fluorochrome est de 5 à 100 µg/ml, de préférence de 10 à 30 µg/ml, plus préférentiellement 20 µg/ml, dans l'échantillon à tester.

Selon un mode de réalisation de l'invention, la concentration finale dans l'échantillon de la vancomycine couplée à un fluorochrome est de 0,15 à 4 µg/ml, de préférence 2 µg/ml, dans l'échantillon à tester.

Selon un mode de réalisation de l'invention, la vancomycine non marquée est incluse pendant l'étape d'incubation a). Dans ce cas, la vancomycine non-marquée et la vancomycine couplée à un fluorochrome peuvent être présentes à un ratio entre 0,25 et 2,25, de préférence à un ratio de 1. Selon un mode de réalisation de l'invention, la vancomycine non-marquée et la vancomycine couplée à un fluorochrome peuvent être présentes à une concentration de 0,15 à 4 µg/ml dans l'échantillon à tester. Selon un mode de réalisation de l'invention, la vancomycine non-marquée et la vancomycine couplée à un fluorochrome peuvent être présentes à une concentration de 2 µg/ml chacun dans l'échantillon à tester.

De manière générale, l'incubation se fait dans des conditions standard connues de l'homme de métier. Par exemple, l'échantillon est incubé dans le noir avec les agents de marquage pendant 10 à 30 minutes, de préférence pendant 15 minutes à température ambiante. L'homme du métier sait que le temps d'incubation peut être plus long si la température d'incubation est plus basse, et plus court si la température d'incubation est plus élevée.

L'étape b) de centrifugation s'effectue selon des conditions habituelles connues de l'homme du métier. Selon un mode de réalisation de l'invention, l'étape b) de centrifugation se fait de 3200g à 15 000g pendant 5-20 minutes.

Selon un mode de réalisation de l'invention, l'étape c) de lavage est effectuée avec une solution tamponnée saline communément appelée PBS (Phosphate Buffer Saline) ou une solution de chlorure de sodium à environ 0,9%.

Selon un mode de réalisation de l'invention, l'étape d) de mesure de la fluorescence de l'échantillon se fait avec un cytomètre en flux. Comme fournisseur d'appareil de ce type, on peut mentionner le cytomètre Influx de BD Biosciences (New Jersey, Etats Unis) et le cytomètre BD Accuri^{™} C6 de BD Biosciences (New Jersey, Etats Unis).

Typiquement, le cytomètre est équipé d'un laser 488 nm (puissance de 200 mW), 405 nm (puissance de 100 mW) et 640 nm (puissance de 120 mW). Le laser de longueur d'onde 488 nm est utilisé pour exciter la vancomycine et sa fluorescence est collectée à travers la bande passante 540/30 nm (540 ± 15nm). Le WGA est excité avec le laser 640 nm et sa fluorescence est collectée à travers la bande passante 670/30 nm (670 ± 15nm). Avant passage d'échantillons, l'appareil peut être calibré pour assurer la meilleure qualité des résultats. Typiquement, un débit de passage des échantillons peut se situer entre 500 événements par seconde et 5000 évènements par seconde. Des enregistrements d'environ 40 000 à 60 000 évènements peuvent être réalisés. L'homme du métier sait que le nombre d'événements peut varier selon le cytomètre utilisé. Selon un mode de réalisation de l'invention, la concentration des bactéries marquées est d'environ 10⁵ à 10⁷, de préférence d'environ 10⁶ ufc/mL.

Les Exemples 1 et 2 et les Figures 1 et 2 démontrent l'efficacité de deux modes de réalisation de l'invention utilisant de la vancomycine couplée à Bodipy^{®} FL et WGA couplé à Alexa Fluor^{®} 647 sans ajout de KCI (Exemple 1) et avec ajout de 1M KCI (Exemple 2). L'ajout de KCI permet d'améliorer le marquage de *Bacillus subtilis.* Dans les deux exemples, les espèces à Gram négatif ne sont pas marquées et les espèces à Gram positif sont bien marquées.

En analysant la fluorescence relative des deux fluorochromes, les inventeurs ont constaté que des familles, des genres, et/ou des espèces bactérien(ne)s différentes avaient leur propre signature de fluorescence spécifique. Ainsi, l'invention a également pour objet un procédé de détection et éventuellement de distinction et d'identification dans un échantillon contenant des bactéries, des familles, des genres, et/ou des espèces de bactéries à Gram positif spécifiques.

Le procédé comprend les étapes décrites ci-dessus, mais également une étape e) d'analyse de la fluorescence relative des deux fluorochromes.

Typiquement, le procédé comprend les étapes suivantes :
a) incubation de l'échantillon, simultanément ou séquentiellement, avec :
   - la lectine du blé (le WGA), couplée à un fluorochrome,
   - la vancomycine couplée à un fluorochrome émettant une fluorescence à une longueur d'onde différente de celle couplée au WGA,
   - optionnellement la vancomycine,
   - optionnellement du chlorure de potassium,
b) centrifugation de l'échantillon,
c) optionnellement lavage de l'échantillon,
d) mesure de la fluorescence de l'échantillon,
e) analyse des fluorescences relatives des deux fluorochromes
f) détection et éventuellement distinction et identification des familles,
genres et/ou espèces bactérien(ne)s à Gram positif et à Gram négatif.

Selon un mode de réalisation dans l'étape f), on distingue et identifie des familles, genres et/ou espèces bactérien(ne)s à Gram positif et à Gram négatif. Selon un mode de réalisation de l'invention, une étape de fixation optionnelle avec, par exemple, une solution de para-formaldéhyde appropriée, peut avoir lieu entre l'étape c) et l'étape d), ou avant l'étape a).

L'étape e) d'analyse peut être effectuée avec le logiciel approprié, par exemple FlowJo^{©} (FlowJo LLC, Ashland, Oregon, Etats Unis).

L'analyse des fluorescences relatives des deux fluorochromes permet de distinguer des genres entre eux, des espèces entre elles et même des souches entre elles. Ceci est possible indépendamment de la viabilité de l'échantillon analysé et également indépendamment du fait que les bactéries soient aérobies ou anaérobies.

Selon un mode de réalisation de l'invention, la signature de fluorescence obtenue permettra non seulement de distinguer, mais aussi d'identifier les familles, genres et/ou espèces présent(e)s dans l'échantillon. Cela est possible en comparant les signatures obtenues de l'échantillon avec des signatures des espèces de référence.

Selon un mode de réalisation de l'invention, l'analyse des fluorescences relatives des deux fluorochromes permet de distinguer des familles bactériennes à Gram positif entre elles car chaque famille aura une signature de fluorescence qui lui est propre.

Par exemple, on peut distinguer les familles suivantes entre elles : *Bacillaceae, Enterococcaceae, Lactobacillaceae, Streptococcaceae, Bifidobacteriaceae Ruminococcaceae, Clostridiaceae, Lachnospiraceae.* Cette liste n'est pas exhaustive.

Selon un mode de réalisation de l'invention, l'analyse des fluorescences relatives des deux fluorochromes permet de distinguer des genres bactériens à Gram positif entre eux car chaque genre aura une signature de fluorescence qui lui est propre.

Par exemple, on peut distinguer les genres suivants entre eux : *Bacillus, Lactococcus, Staphylococcus, Bifidobacterium, Enterococcus, Lactobacillus, Clostridium, Blautia, Roseburia, Eubacterium, Anaerostipes* et *Faecalibacterium* par exemple. Cette liste n'est pas exhaustive.

L'invention permet de distinguer des bactéries à Gram positif anaérobies des bactéries à Gram négatif anaérobies. Elle permet aussi de distinguer des bactéries à Gram positif aérobies des bactéries à Gram négatif aérobies. Comme exemples de bactéries anaérobies, on peut citer :
- *Akkermansia muciniphila*
- *Faecalibacterium prausnitzii*
- *Clostridium scindens*
- *Bacteroides thetaiotamicron*
- Et celles appartenant aux *Clostridiales.*

Selon un mode de réalisation de l'invention, l'analyse des fluorescences relatives des deux fluorochromes permet de distinguer des espèces bactériennes à Gram positif et à Gram négatif car chaque espèce aura une signature de fluorescence qui lui est propre. Par exemple, on peut citer des espèces suivantes comme des espèces qu'on peut distinguer entre elles : *Salmonella enterica, Salmonella bongori, Bacillus subtilis, Bacillus cereus, Bacillus thuringiensis, Lactococcus lactis, Lactococcus plantarum, Escherichia coli, Staphylococcus aureus, Staphylococcus epidermidis, Enterococcus faecalis, Enterococcus hirae, Enterococcus durons, Enterococcus casseliflavus, Enterococcus gallinarum, Klebsiella pneumoniae, Lactobacillus crispatus, Lactobacillus reuteri, Lactobacillus gasseri* et *Lactobacillus brevis, Faecalibacterium prausnitzii, Akkermansia muciniphila, Clostridium spiroform, Clostridium innocum, Clostridium scindens, Flavonifractor ploutii, Bacteroides thetaiotamicron, Bacteroides fragilis, Blautia hansenii et Blautia schinkii* par exemple. Cette liste n'est pas exhaustive.

L'Exemple 3 montre comment il est possible, en mettant en œuvre le procédé selon un mode de réalisation, de distinguer au moins six espèces différentes en analysant les fluorescences relatives de deux marqueurs de la vancomycine couplée à Bodipy^{®} FL et du WGA couplé à Alexa Fluor^{®} 647. En comparant des signatures obtenues avec des signatures de références il est possible d'identifier des espèces.

Les Figures 3A et 3B montrent les résultats de l'expérience décrite à l'Exemple 3 où la fluorescence relative des agents de marquage - la vancomycine couplée à Bodipy FL^{®} et le WGA couplé à Alexa Fluor^{®} 647 - est mesurée. Le graphique de la Figure 3A montre la moyenne géométrique des fluorescences obtenues, pour les différentes espèces testées, à une longueur d'onde de 540nm ± 15nm, qui correspond à la fluorescence de vancomycine couplée à Bodipy^{®} FL. On constate que les membres de la même espèce se trouvent groupés ensemble et que l'analyse permet de distinguer des espèces différentes : *Salmonella enterica, Bacillus subtilis, Escherichia coli, Staphylococcus aureus* et *Enterococcus faecalis.* Chaque espèce occupe une zone différente du graphique.

Le graphique de la Figure 3B montre la moyenne géométrique des fluorescences obtenues, pour les différentes espèces testées, à une longueur d'onde de 670nm ± 15nm, qui correspond à la fluorescence de WGA couplé à Alexa Fluor^{®} 647. On constate que les membres de la même espèce sont groupés ensemble, à part l'espèce *E. faecalis* pour laquelle les membres sont plus dispersés. Ainsi, l'analyse avec les deux marqueurs permet de distinguer des espèces bactériennes à Gram positif, comme par exemple, *Bacillus subtilis, Staphylococcus aureus, Enterococcus faecalis et Lactobacillus spp.*

Selon un mode de réalisation de l'invention, l'analyse des fluorescences relatives des deux fluorochromes permet de déterminer le ratio de bactéries à Gram positif sur des bactéries à Gram négatif de cultures *in vitro* de microbiote.

Un back gating peut être effectué sur les populations mises en évidence dans l'analyse des fluorescences relatives des deux fluorochromes choisis. Un « back gating » est une technique utilisée en cytométrie en flux, connue de l'homme de métier, qui permet de confirmer une signature de marquage (et la méthode de « gating » utilisée). Selon un mode de réalisation de l'invention, à partir des populations identifiées sur les graphiques représentant l'intensité de la fluorescence à une longueur d'onde de, par exemple, 540nm ± 15nm, qui correspond à la fluorescence de la vancomycine couplée à Bodipy^{®} FL, et l'intensité de la fluorescence à une longueur d'onde de, par exemple, 670nm ± 15nm, qui correspond à la fluorescence de WGA couplé à Alexa Fluor^{®} 647, le back gating comprendrait la sélection d'une des populations et le retour sur le profil FCS/SSC de cette dernière. La méthode revendiquée permet ainsi de potentiellement identifier certaines populations. Elle permet de mettre en évidence différentes populations au niveau des familles, genres et/ou espèces présent(e)s dans la culture et d'estimer leur diversité, ainsi que le pourcentage de bactéries à Gram négatif et de bactéries à Gram positif.

Selon un mode de réalisation de l'invention, la méthode revendiquée pourrait être utilisée pour corréler les résultats du marquage Gram en cytométrie à ceux d'analyse 16S.

L'efficacité de cette analyse est très utile pour l'analyse des échantillons complexes comme les échantillons de microbiote. L'invention permet ainsi de caractériser des échantillons complexes qui peuvent avoir une utilisation médicale, ou une utilisation en tant qu'outil de recherche, en particulier, en métagénomique. Ainsi, l'importance de la fiabilité, de la reproductibilité, de la précision et de la sensibilité de l'analyse est primordiale.

L'invention est en outre décrite en référence aux exemples suivants. Il sera apprécié que l'invention telle que revendiquée ne soit pas destinée à être limitée de quelque manière que ce soit par ces exemples.

### EXEMPLES

### Exemple 1

Les différentes espèces testées ont été cultivées toute la nuit à 37 ± 1°C ou 30 ± 1°C avec ou sans agitation à 180 rpm dans du milieu Luria Bertani (LB) ou Brain Heart Infusion (BHI) selon les besoins des différentes souches. Les différentes suspensions bactériennes ont été ajustées aux alentours de 10⁶ ufc/mL. Les marquages ont été préparés en amont en combinant la vancomycine couplée à Bodipy^{®} FL, la vancomycine et le WGA couplé à Alexa Fluor^{®} 647 aux concentrations de 0,5 µg/mL, 0,5 µg/mL et 100 µg/mL respectivement. Le marquage a été mis en contact avec les différentes espèces à tester. Après 15 minutes d'incubation dans le noir à température ambiante, le lavage a été effectué avec une solution de PBS suivi d'une étape de centrifugation à 3220 g pendant 15 minutes. Les échantillons ont par la suite été analysés avec le cytomètre Influx (BD).

Le cytomètre Influx (BD), équipé d'un laser 488 nm (puissance de 200 mW), 405 nm (puissance de 100 mW) et 640 nm (puissance de 120 mW), a été utilisé pour les analyses en cytométrie. Les signaux de taille (Forward : FSC) et de granularité (Side : SCC) sont obtenus à 488 nm. Le laser de longueur d'onde 488 nm est utilisé pour exciter la vancomycine et sa fluorescence est collectée à travers la bande passante 540/30 nm. Le WGA est excité avec le laser 640 nm et sa fluorescence est collectée à travers la bande passant 670/30 nm. Avant tout passage d'échantillons, des billes de calibration sont utilisées pour réaliser le contrôle qualité de la calibration du cytomètre. Le débit de passage des échantillons a été maintenu entre 500 évènements par seconde et 5000 événements par seconde. Des enregistrements de 50 000 évènements ont été réalisés.

Les analyses ont été effectuées avec le logiciel FlowJo. Le bruit de fond a été éliminé pour les analyses des marquages Gram et le ciblage des populations Gram a été effectué manuellement autour des groupes de populations visibles.

### Résultats

La Figure 1 montre les résultats de l'expérience décrite à l'Exemple 1 dans lequel des échantillons de deux espèces à Gram négatif (*Klebsiella pneumoniae* ATCC 700603 et *Salmonella enterica* ATCC 13311) et de deux espèces à Gram positif (*Enterococcusfaecalis* JH2.2 et Lactococcus lactis MG1363) sont analysés selon un mode de réalisation de l'invention.

Sur les graphiques, l'axe X représente l'intensité de la fluorescence à une longueur d'onde de 540nm ± 15nm, qui correspond à la fluorescence de la vancomycine couplée à Bodipy FL^{®} et l'axe Y représente l'intensité de la fluorescence à une longueur d'onde de 670nm ± 15nm, qui correspond à la fluorescence de WGA couplé à Alexa Fluor^{®} 647.

On constate que les espèces à Gram négatif ne sont pas marquées et que les espèces à Gram positif sont bien marquées.

### Exemple 2

Les différentes espèces testées ont été cultivées toute la nuit à 37 ± 1°C ou 30 ± 1°C avec ou sans agitation à 180 rpm dans du milieu Luria Bertani (LB) ou Brain Heart Infusion (BHI) selon les besoins des différentes souches. Les différentes suspensions bactériennes ont été ajustées aux alentours de 10⁶ ufc/mL soit en PBS (pour la condition PBS) soit en KCI 1M (pour la condition KCI). Certaines suspensions bactériennes ont été traitées au para-formaldéhyde (PFA) 1% pendant 1h à température ambiante, suivies de deux centrifugations à 3220 g pendant 15 minutes pour enlever tous résidus de PFA. Les espèces bactériennes ont soit été suspendues en PBS soit en KCI 1M selon la condition testée et les marquages, préalablement préparés, combinant la vancomycine couplée à Bodipy^{®} FL, la vancomycine et le WGA couplé à Alexa Fluor^{®} 647 aux concentrations de 2 µg/mL, 2 µg/mL et 20 µg/mL respectivement ont été mis en contact avec les suspensions bactériennes. Après 15 minutes d'incubation des marquages dans le noir à température ambiante et un lavage avec une solution de PBS et puis une centrifugation à 3220 g pendant 15 minutes, les échantillons ont été analysés avec le cytomètre Influx (BD).

### Résultats

La Figure 2 montre les résultats de l'expérience décrite à l'Exemple 2 dans lequel des échantillons de trois espèces à Gram négatif (*Klebsiella pneumoniae* ATCC 700603, *Salmonella enterica* ATCC 13311 et *E. coli* ATCC 35218) et de trois espèces à Gram positif (*Enteroccus faecalis* JH2.2 et *Lactococcus lactis* MG1363 et *Bacillus subtilis* ATCC 23857) sont analysés selon un mode de réalisation de l'invention. Les bactéries ont été marquées avec du WGA couplé à Alexa Fluor^{®} 647 et de la vancomycine couplée à Biopdy FL^{®} en présence de 1M KCl. On constate que les espèces à Gram négatif ne sont pas marquées et que les trois espèces à Gram positif sont bien marquées.

### Exemple 3

Les différentes espèces testées ont été cultivées toute la nuit à 37 ± 1°C ou 30 ± 1°C avec ou sans agitation à 180 rpm dans du milieu Luria Bertani (LB) ou Brain Heart Infusion (BHI) selon les besoins des différentes souches. Les différentes suspensions bactériennes ont été ajustées aux alentours de 10^{E}6 ufc/mL. Les marquages ont été préparés en amont en combinant la vancomycine couplée à Bodipy^{®} FL, la vancomycine et le WGA couplé à Alexa Fluor^{®} 647 aux concentrations de 2 µg/mL, 2 µg/mL et 20 µg/mL respectivement dans une solution contenant du KCI à 1M final. Le marquage a été mis en contact avec les différentes espèces à tester. Après 15 minutes d'incubation dans le noir à température ambiante, le lavage a été effectué avec une solution de PBS suivi d'une étape de centrifugation à 15000 g pendant 5 minutes. Les échantillons ont par la suite été analysés avec le cytomètre Influx (BD).

## Revendications

1. Procédé de détection dans un échantillon contenant des bactéries, de la proportion de bactéries à Gram positif et de la proportion de bactéries à Gram négatif, comprenant les étapes :
a) incubation de l'échantillon, simultanément ou séquentiellement, avec :
- la lectine du blé (le WGA), couplée à un fluorochrome,
- la vancomycine couplée à un fluorochrome émettant une fluorescence à une longueur d'onde différente de celle couplée au WGA,
- optionnellement la vancomycine non-couplée à un fluorochrome (non-marquée),
- optionnellement du chlorure de potassium.
b) centrifugation de l'échantillon,
c) optionnellement lavage de l'échantillon,
d) mesure de la fluorescence de l'échantillon en cytométrie en flux.

2. Procédé selon la revendication 1, **caractérisé en ce que** le fluorochrome couplé au WGA est Alexa Fluor^{®} 647 et celui couplé à la vancomycine est l'acide propénoïque 3-BODIPY.

3. Procédé selon la revendication 1 ou 2, **caractérisé en ce que** l'étape de mesure est effectuée en moins de 40 minutes, de préférence, en moins de 30 minutes après la fin de l'étape c).

4. Procédé selon la revendication 1, 2 ou 3, **caractérisé en ce que** l'étape d) de mesure est effectuée avec un cytomètre en flux.

5. Le procédé selon l'une des revendications 1 à 4, **caractérisé en ce que**, à l'étape a), la concentration de la lectine du blé (le WGA) couplée à un fluorochrome, dans l'échantillon à analyser, est de 5 à 100 µg/ml, de préférence de 20 µg/ml et/ou la concentration finale de la vancomycine couplée à un fluorochrome, dans l'échantillon à analyser, est de 0,15 à 4 µg/ml, de préférence 2 µg/ml.

6. Le procédé selon l'une des revendications 1 à 5, **caractérisé en ce que** le temps d'incubation de l'étape a) est de 10 à 30 minutes, de préférence de 15 minutes.

7. Procédé selon l'une des revendications 1 à 6, **caractérisé en ce que** l'échantillon est un échantillon de microbiote humain ou animal.

8. Procédé de détection, éventuellement de distinction et d'identification dans un échantillon bactérien des familles, genres et/ou des espèces de bactéries à Gram positif et de bactéries à Gram négatif comprenant les étapes :
a) incubation de l'échantillon, simultanément ou séquentiellement, avec :
- la lectine du blé (le WGA), couplée à un fluorochrome,
- la vancomycine couplée à un fluorochrome émettant une fluorescence à une longueur d'onde différente de celle couplée au WGA,
- optionnellement la vancomycine,
- optionnellement du chlorure de potassium,
b) centrifugation de l'échantillon,
c) optionnellement lavage de l'échantillon,
d) mesure de la fluorescence de l'échantillon en cytométrie en flux,
e) analyse des fluorescences relatives des deux fluorochromes,
f) éventuellement distinction et identification des familles, genres et/ou espèces bactérien(ne)s à Gram positif et à Gram négatif.

9. Procédé selon la revendication 8 **caractérisé en ce que** le fluorochrome couplé au WGA est Alexa Fluor^{®} 647 et celui couplé à la vancomycine est l'acide propénoïque 3-BODIPY.

10. Utilisation d'un kit comprenant :
i. la lectine du blé (le WGA), couplée à un fluorochrome,
ii. la vancomycine couplée à un fluorochrome émettant une fluorescence à une longueur d'onde différente de celle couplée au WGA, et
iii. optionnellement la vancomycine,
iv. optionnellement du chlorure de potassium,
pour le marquage des bactéries à Gram positif en cytométrie en flux.

11. L'utilisation selon la revendication 10, **caractérisée en ce que** les fluorochromes sont choisis parmi le groupe des agents de marquage Alexa Fluor^{®}, l'acide propénoïque 3-BODIPY ou un de ses dérivés, la fluorescéine ou l'un de ses dérivés, sels ou esters, les agents de marquages Cy, les agents de marquages CF, des fluorochromes conjugués.

12. L'utilisation selon la revendication 11, **caractérisée en ce que** le fluorochrome couplé au WGA est choisi parmi des agents de marquage Alexa Fluor^{®}, la fluorescéine ou l'un de ses dérivés, sels ou esters.

13. L'utilisation selon la revendication 11, **caractérisée en ce que** le fluorochrome couplé à la vancomycine est choisi parmi l'acide propénoïque 3-BODIPY ou un de ses dérivés, la fluorescéine ou l'un de ses dérivés, sels ou esters, notamment FITC.

14. L'utilisation selon l'une des revendications 11 à 13, **caractérisée en ce que** le fluorochrome couplé au WGA est Alexa Fluor^{®} 647 et celui couplé à la vancomycine est l'acide propénoïque 3-BODIPY.

## Patentansprüche

1. Verfahren zum Nachweisen des Anteils grampositiver Bakterien und des Anteils gramnegativer Bakterien in einer Bakterien enthaltenden Probe, welches die Schritte umfasst:
a) Inkubieren der Probe, gleichzeitig oder nacheinander, mit:
- Weizenlektin (WGA), gekoppelt an ein Fluorochrom,
- Vancomycin, gekoppelt an ein Fluorochrom, das eine Fluoreszenz bei einer Wellenlänge emittiert, die von derjenigen verschieden ist, die an WGA gekoppelt ist,
- optional Vancomycin, das nicht an ein Fluorochrom gekoppelt (unmarkiert) ist,
- optional Kaliumchlorid.
b) Zentrifugieren der Probe,
c) optional Waschen der Probe,
d) Messung der Fluoreszenz der Probe in Durchflusszytometrie.

2. Verfahren nach Anspruch 1, **dadurch gekennzeichnet, dass** das an WGA gekoppelte Fluorochrom Alexa Fluor^{®} 647 ist und dasjenige, das an Vancomycin gekoppelt ist, 3-BODIPY-Propensäure ist.

3. Verfahren nach Anspruch 1 oder 2, **dadurch gekennzeichnet, dass** der Messungs-Schritt innerhalb von weniger als 40 Minuten, bevorzugt innerhalb von weniger als 30 Minuten nach dem Ende des Schrittes c) durchgeführt wird.

4. Verfahren nach Anspruch 1, 2 oder 3, **dadurch gekennzeichnet, dass** der Messungs-Schritt d) mit einem Durchflusszytometer durchgeführt wird.

5. Verfahren nach einem der Ansprüche 1 bis 4, **dadurch gekennzeichnet, dass** im Schritt a) die Konzentration des an ein Fluorochrom gekoppelten Weizenlektins (WGA) in der zu analysierenden Probe 5 bis 100 µg/ml beträgt, bevorzugt 20 µg/ml, und/oder die Endkonzentration des an ein Fluorochrom gekoppelten Vancomycins in der zu analysierenden Probe 0,15 bis 4 µg/ml beträgt, bevorzugt 2 µg/ml.

6. Verfahren nach einem der Ansprüche 1 bis 5, **dadurch gekennzeichnet, dass** die Inkubationsdauer des Schrittes a) 10 bis 30 Minuten beträgt, bevorzugt 15 Minuten.

7. Verfahren nach einem der Ansprüche 1 bis 6, **dadurch gekennzeichnet, dass** die Probe eine menschliche oder tierische Mikrobiota-Probe ist.

8. Verfahren zum Nachweisen, gegebenenfalls Unterscheiden und Identifizieren, von Familien, Gattungen und/oder Arten grampositiver Bakterien und gramnegativer Bakterien, welches die Schritte umfasst:
a) Inkubieren der Probe, gleichzeitig oder nacheinander, mit:
- Weizenlektin (WGA), gekoppelt an ein Fluorochrom,
- Vancomycin, gekoppelt an ein Fluorochrom, das eine Fluoreszenz bei einer Wellenlänge emittiert, die von derjenigen verschieden ist, die an WGA gekoppelt ist,
- optional Vancomycin,
- optional Kaliumchlorid,
b) Zentrifugieren der Probe,
c) optional Waschen der Probe,
d) Messung der Fluoreszenz der Probe in Durchflusszytometrie,
e) Analyse der relativen Fluoreszenzen der zwei Fluorochrome,
f) gegebenenfalls Unterscheidung und Identifizierung der grampositiven und gramnegativen Bakterien-Familien, -Gattungen und/oder -Arten.

9. Verfahren nach Anspruch 8, **dadurch gekennzeichnet, dass** das an WGA gekoppelte Alexa Fluor^{®} 647 ist und dasjenige, das an Vancomycin gekoppelt ist, 3-BODIPY-Propensäure ist.

10. Verwendung eines Kits, welches umfasst:
i. Weizenlektin (WGA), gekoppelt an ein Fluorochrom,
ii. Vancomycin, gekoppelt an ein Fluorochrom, das eine Fluoreszenz bei einer Wellenlänge emittiert, die von derjenigen verschieden ist, die an WGA gekoppelt ist, und
iii. optional Vancomycin,
iv. optional Kaliumchlorid,
zum Markieren grampositiver Bakterien in Durchflusszytometrie.

11. Verwendung nach Anspruch 10, **dadurch gekennzeichnet, dass** die Fluorochrome aus der Gruppe von Alexa-Fluor^{®}-Markierungsmitteln, 3-BODIPY-Propensäure oder einem ihrer Derivate, Fluorescein oder einem seiner Derivate, Salze oder Ester, Cy-Markierungsmitteln, CF-Markierungsmitteln, konjugierten Fluorochromen ausgewählt sind.

12. Verwendung nach Anspruch 11, **dadurch gekennzeichnet, dass** das an WGA gekoppelte Fluorochrom aus Alexa-Fluor^{®}-Markierungsmitteln, Fluorescein oder einem seiner Derivate, Salze oder Ester ausgewählt ist.

13. Verwendung nach Anspruch 11, **dadurch gekennzeichnet, dass** das an Vancomycin gekoppelte Fluorochrom aus 3-BODIPY-Propensäure oder einem ihrer Derivate oder Fluorescein oder einem seiner Derivate, Salze oder Ester, insbesondere FITC, ausgewählt ist.

14. Verwendung nach einem der Ansprüche 11 bis 13, **dadurch gekennzeichnet, dass** das an WGA gekoppelte Fluorochrom Alexa Fluor^{®} 647 ist und dasjenige, das an Vancomycin gekoppelt ist, 3-BODIPY-Propensäure ist.

## Claims

1. Method for detecting, in a sample containing bacteria, the proportion of Gram-positive bacteria and the proportion of Gram-negative bacteria, comprising the steps of:
a) incubating the sample, simultaneously or sequentially, with:
- wheat lectin (WGA) coupled to a fluorochrome,
- vancomycin coupled to a fluorochrome emitting a fluorescence at a different wavelength from that coupled to WGA,
- optionally vancomycin that is not coupled to a fluorochrome (unlabelled),
- optionally potassium chloride,
b) centrifuging the sample,
c) optionally washing the sample,
d) measuring the fluorescence of the sample using flow cytometry.

2. Method according to claim 1, **characterized in that** the fluorochrome coupled to WGA is Alexa Fluor^{®} 647 and the one coupled to vancomycin is 3-BODIPY-propanoic acid.

3. Method according to claim 1 or 2, **characterized in that** the measurement step is carried out in less than 40 minutes, preferably in less than 30 minutes, after the end of step c).

4. Method according to claim 1, 2 or 3, **characterized in that** the measurement step d) is carried out with a flow cytometer.

5. Method according to one of claims 1 to 4, **characterized in that**, in step a), the concentration of the wheat lectin (WGA) coupled to a fluorochrome in the sample to be analysed is from 5 to 100 µg/ml, preferably 20 µg/ml, and/or the final concentration of the vancomycin coupled to a fluorochrome in the sample to be analysed is from 0.15 to 4 µg/ml, preferably 2 µg/ml.

6. Method according to one of claims 1 to 5, **characterized in that** the incubation time in step a) is from 10 to 30 minutes, preferably 15 minutes.

7. Method according to one of claims 1 to 6, **characterized in that** the sample is a sample of human or animal microbiota.

8. Method for detecting, optionally distinguishing and identifying families, genera and/or species of Gram-positive bacteria and Gram-negative bacteria in a bacterial sample, comprising the steps of:
a) incubating the sample, simultaneously or sequentially, with:
- wheat lectin (WGA) coupled to a fluorochrome,
- vancomycin coupled to a fluorochrome emitting a fluorescence at a different wavelength from that coupled to WGA,
- optionally vancomycin,
- optionally potassium chloride,
b) centrifuging the sample,
c) optionally washing the sample,
d) measuring the fluorescence of the sample using flow cytometry,
e) analysing the relative fluorescences of the two fluorochromes,
f) optionally distinguishing and identifying families, genera and/or species of Gram-positive and Gram-negative bacteria.

9. Method according to claim 8, **characterized in that** the fluorochrome coupled to WGA is Alexa Fluor^{®} 647 and the one coupled to vancomycin is 3-BODIPY-propanoic acid.

10. Use of a kit comprising:
i. wheat lectin (WGA) coupled to a fluorochrome,
ii. vancomycin coupled to a fluorochrome emitting a fluorescence at a different wavelength from that coupled to WGA, and
iii. optionally vancomycin,
iv. optionally potassium chloride,
for labelling Gram-positive bacteria in flow cytometry.

11. Use according to claim 10, **characterized in that** the fluorochromes are selected from the group of labelling agents Alexa Fluor^{®}, 3-BODIPY-propanoic acid or one of its derivatives, fluorescein or one of its derivatives, salts or esters, Cy labelling agents, CF labelling agents, conjugated fluorochromes.

12. Use according to claim 11, **characterized in that** the fluorochrome coupled to WGA is selected from the Alexa Fluor^{®} labelling agents, fluorescein or one of its derivatives, salts or esters.

13. Use according to claim 11, **characterized in that** the fluorochrome coupled to vancomycin is selected from 3-BODIPY-propanoic acid or one of its derivatives, fluorescein or one of its derivatives, salts or esters, in particular FITC.

14. Use according to one of claims 11 to 13, **characterized in that** the fluorochrome coupled to WGA is Alexa Fluor^{®} 647 and the one coupled to vancomycin is 3-BODIPY-propanoic acid.
